# EUROPEAN PATENT APPLICATION

(11) **EP 4 732 841 A1**
(43) Date of publication of application: **29.04.2026**
(21) Application number: 24838597.3
(22) Date of filing: 26.06.2024
(51) Int. Cl.: A61K 31/77, A61K 9/08, A61P 7/04, A61P 35/00, A61P 31/00, A61P 29/00

(54) **FOAM SCLEROSANT COMPOSITION, AND PREPARATION METHOD THEREFOR AND USE THEREOF**

(30) Priority: 13.07.2023 CN 202310858307
(71) Applicant: Jenkem Technology Co., Ltd. (Beijing), Haidian District Beijing 100192 (CN)
(72) Inventor: WANG, Qingbin, Beijing 100192 (CN); LIU, Biao, Beijing 100192 (CN); ZHENG, Xichun, Beijing 100192 (CN); LI, Qiufen, Beijing 100192 (CN); WANG, Jie, Beijing 100192 (CN); GUO, Jun, Beijing 100192 (CN); ZHAO, Xuan, Beijing 100192 (CN)
(74) Representative: HGF
(86) International application number: PCT/CN2024/101673
(87) International publication number: WO 2025/011341

(57) **Abstract**

A foam sclerosant composition, and a preparation method therefor and a use thereof. The foam sclerosant composition comprises polidocanol with a single molecular weight and sodium hyaluronate in a volume ratio of 1:0.1-5. A preparation method for polidocanol with a single molecular weight comprises the following steps: reacting polyethyleneglycol with a single molecular weight with 1-halododecane, and carrying out separation and purification, thereby obtaining polidocanol with a single molecular weight. The foam sclerosant has higher foam stability, better drug effects, and reduced toxic and side effects, and can be widely used for treating vascular related diseases, cystic diseases, tumors, gynecological and obstetrical diseases, inflammations or pains.

## Description

### TECHNICAL FIELD

The present disclosure pertains to the technical field of pharmaceutical formulations, and particularly relates to a foam sclerosant composition, a preparation method therefor, and use thereof.

### BACKGROUND

Polidocanol (POL) is a surfactant-based sclerosant. Its structure exhibits high similarity to the phospholipid bilayer of the cell membrane. By interfering with surface active substances on the cell membrane, it disrupts the cell membrane structure, leading to lysis and death of vascular endothelial cells, thereby causing vascular fibrosis and vascular occlusion. Simultaneously, polidocanol possesses fixed hydrophilic and hydrophobic ends, enabling oriented arrangement at the solution surface, so that the surface tension of the solution is significantly reduced, and the foam formation can be achieved. Therefore, polidocanol is widely used in foam sclerotherapy. Currently, polidocanol is primarily applied in sclerotherapy for varicose veins, venous malformations, hemangiomas, internal hemorrhoids, cystic diseases, acne, tumor ablation, and the like. In sclerotherapy, the therapeutic efficiency of foam sclerosants depends on foam stability and the uniformity of bubble size. Generally, the smaller the average bubble diameter in the foam, the more uniform the foam, the higher the drug concentration, the better the foam stability, and the better the therapeutic effect. Typically, indicators such as foam fineness, liquid drainage time, and half-life are used to evaluate foam stability, which is influenced by various factors, including the composition of the sclerosant, liquid viscosity, and surface properties of the liquid.

Liu Ziyi et al. disclosed a hyaluronic acid-polidocanol composition and investigated the effects of hyaluronic acid (HA) on the vascular endothelial cell-killing ability, foam stability, blood displacement capacity, and viscosity of polidocanol foam. The research results showed that HA can promote endothelial cell proliferation, but 1% POL exhibits a stronger cell-killing ability. When both act on endothelial cells simultaneously, the cells undergo immediate lysis and death. HA can significantly increase the viscosity of POL foam, thereby enhancing foam stability and blood displacement capacity. Within a certain range (adding HA at a dose of ≤0.4 mL to 1 mL of POL), the greater the dose of HA added to POL, the better the foam performance (see: Liu Ziyi. The role of hyaluronic acid in polidocanol foam: An in vitro study [D]. Shandong University, 2022.). However, this study used commercially available polidocanol, which is a mixture containing different molecular weights and numerous impurities. Its drug effect is influenced by polidocanol of different chain lengths and impurities, making it difficult to control during application.

Polidocanol, also known as aethoxysklerol or lauromacrogol, is a polymer product obtained by polymerizing lauryl alcohol with multiple ethylene oxide molecules. Currently, polidocanol is primarily prepared by the polymerization of lauryl alcohol and ethylene oxide under alkaline catalysis. The quality and stability of polidocanol products are significantly affected by the purity of starting materials, catalysts, reaction pressure, temperature, post-treatment processes, and the like. In one aspect, commercially available lauryl alcohol contains numerous impurities, including decanol and tetradecanol, as well as many unknown impurities. The by-products generated after these impurities are introduced into the polidocanol polymerization reaction are difficult to separate from polidocanol, which directly impacts the quality of the finished polidocanol product. In another aspect, in addition to unreacted lauryl alcohol and ethylene oxide, the synthesis of polidocanol produces many by-products, mainly including free polyethylene glycol, diethylene glycol, dioxane, formaldehyde, acetaldehyde, ethylene glycol, and the like. These impurities significantly adversely affect the safety and efficacy of polidocanol.

The patent with publication number CN103922901 discloses a purification method for polidocanol, which involves distillation under reduced pressure before adjusting the pH of the crude product to ultimately obtain a high-purity refined polidocanol product. However, this invention cannot avoid the risk of by-products formed from impurities (such as n-decanol and n-tetradecanol) introduced by lauryl alcohol reacting with ethylene oxide.

The patent with publication number CN113200821A discloses a purification method for lauryl alcohol and a synthesis method for polidocanol, which prepares high-purity lauryl alcohol through rectification under reduced pressure. However, the rectification purification requires -0.08 MPa and a rectification temperature of 130-150 °C. In the process of preparing polidocanol, the dehydration temperature for lauryl alcohol is 90-125 °C, the vacuum degree is -0.08 MPa, the polymerization temperature is 100-115 °C, the polymerization pressure is 0.1-0.8 MPa, and after the polymerization is completed, the degassing temperature is 90-120 °C. It can be seen that the polymerization reaction proceeds under high temperature and negative pressure conditions, imposing high requirements on equipment and processes.

Currently, commercially available polidocanol is a mixture of different molecular weights. Polidocanol having different chain lengths exhibits varying foaming effects and drug effects, making it difficult to control during application. Therefore, the research, development, and preparation of polidocanol with a single molecular weight to effectively control and improve the quality of polidocanol are of significant importance for enhancing the drug effect of polidocanol foam sclerosants and compositions thereof.

### SUMMARY

In order to solve the problems described above, the present disclosure provides a foam sclerosant composition, a preparation method therefor, and use thereof, wherein the foam sclerosant composition is a combination of sodium hyaluronate and polidocanol with a single molecular weight.

In a first aspect of the present disclosure, provided is a foam sclerosant composition, and the foam sclerosant composition comprises polidocanol and sodium hyaluronate, wherein the polidocanol is polidocanol with a single molecular weight, and the monomer molecular structure thereof comprises the following structure: 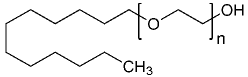
wherein n is an integer, and 4 ≤ n ≤ 24;
specifically, n is selected from any one of the values in 4, 5, 6, 7, 8, 9, 10, 11, 12, 13, 14, 15, 16, 17, 18, 19, 20, 21, 22, 23, and 24, and each value of n corresponds to one type of polidocanol with a definite molecular weight.

Preferably, 8 ≤ n ≤ 15;
more preferably, n is 8, 9, or 10.

Further, the volume ratio of the polidocanol with a single molecular weight to the sodium hyaluronate is in the range of 1:0.1 to 1:5 (specifically, such as 1:0.1, 1:0.2, 1:0.3, 1:0.4, 1:0.5, 1:0.6, 1:0.7, 1:0.8, 1:0.9, 1:1, 1:1.1, 1:1.2, 1:1.3, 1:1.4, 1:1.5, 1:1.6, 1:1.7, 1:1.8, 1:1.9, 1:2, 1:2.2, 1:2.4, 1:2.6, 1:2.8, 1:3, 1:3.2, 1:3.4, 1:3.6, 1:3.8, 1:4, 1:4.2, 1:4.4, 1:4.6, 1:4.8, or 1:5), preferably in the range of 1:0.5 to 1:2, and more preferably 1:1.

Further, the mass concentration of the polidocanol with a single molecular weight is 0.01-5% (specifically, such as 0.01%, 0.025%, 0.05%, 0.075%, 0.1%, 0.125%, 0.15%, 0.2%, 0.25%, 0.3%, 0.35%, 0.4%, 0.45%, 0.5%, 1%, 1.5%, 2%, 2.5%, 3%, 3.5%, 4%, 4.5%, or 5%), preferably 0.05-2%, and more preferably 1%.

Further, the mass concentration of the sodium hyaluronate is 0.01-5% (specifically, such as 0.01%, 0.025%, 0.05%, 0.075%, 0.1%, 0.125%, 0.15%, 0.2%, 0.25%, 0.3%, 0.35%, 0.4%, 0.45%, 0.5%, 0.75%, 1%, 1.25%, 1.5%, 1.75%, 2%, 2.25%, 2.5%, 2.75%, 3%, 3.25%, 3.5%, 3.75%, 4%, 4.25%, 4.5%, 4.75%, or 5%), preferably 0.05-2%, and more preferably 0.05-0.25%.

Further, the foam sclerosant composition further comprises a pharmaceutically acceptable excipient. Further, the pharmaceutically acceptable excipient is selected from one or more of a solubilizer, an antioxidant, an anti-photolysis agent, a pH regulator, and an emulsifier.

Further, the dosage form of the foam sclerosant composition is an injection.

In a second aspect of the present disclosure, provided is a preparation method for the foam sclerosant composition according to the first aspect, and the preparation method comprises the following steps:
(1) preparation of polidocanol with a single molecular weight: reacting polyethylene glycol with a single molecular weight with 1-halododecane, separating, and purifying, thereby obtaining the polidocanol with a single molecular weight;
(2) preparation of a foam sclerosant composition: dissolving the polidocanol with a single molecular weight described above in a solvent, allowing the solution to stand, adding sodium hyaluronate, mixing the mixture homogeneously to obtain a mixed solution, and mixing the mixed solution described above with a gas to generate foam, thereby obtaining the foam sclerosant composition. Further, in step (1), the preparation method for the polidocanol with a single molecular weight comprises the following steps:
   A1: mixing polyethylene glycol with a single molecular weight with a catalyst and an organic solvent, adding 1-halododecane dropwise, heating to a preset temperature, and performing reaction with the temperature maintained;
   A2: after the reaction is completed, cooling the reactant, and after the reactant is cooled to room temperature, adding water for dilution, followed by extraction and liquid separation to collect an organic phase;
   A3: filtering, concentrating, drying, separating, and purifying the organic phase to obtain the polidocanol with a single molecular weight.

Further, in step A1, the polyethylene glycol with a single molecular weight is selected from tetraethylene glycol, pentaethylene glycol, hexaethylene glycol, heptaethylene glycol, octaethylene glycol, nonaethylene glycol, decaethylene glycol, undecaethylene glycol, dodecaethylene glycol, tridecaethylene glycol, tetradecaethylene glycol, pentadecaethylene glycol, hexadecaethylene glycol, heptadecaethylene glycol, octadecaethylene glycol, nonadecaethylene glycol, eicosaethylene glycol, heneicosaethylene glycol, docosaethylene glycol, tricosaethylene glycol, and tetracosaethylene glycol; preferably, the polyethylene glycol with a single molecular weight is octaethylene glycol, nonaethylene glycol, decaethylene glycol, undecaethylene glycol, dodecaethylene glycol, tridecaethylene glycol, tetradecaethylene glycol, or pentadecaethylene glycol; more preferably, the polyethylene glycol with a single molecular weight is octaethylene glycol, nonaethylene glycol, or decaethylene glycol.

Further, in step A1, the catalyst is selected from sodium hydroxide, sodium hydride, potassium hydroxide, potassium hydride, potassium tert-butoxide, and sodium tert-butoxide, and preferably, the catalyst is sodium hydroxide and/or sodium hydride.

Further, in step A1, the organic solvent is selected from one or more of ethanol, methanol, isopropanol, tetrahydrofuran (THF), acetone, acetonitrile, toluene, xylene, n-hexane, cyclohexane, isooctane, n-pentane, petroleum ether, dimethyl sulfoxide (DSMO), N,N-dimethylformamide, dichloromethane (DCM), and diethoxymethane (DEM), and preferably, the organic solvent is one or more of tetrahydrofuran (THF), dimethyl sulfoxide (DSMO), acetonitrile, toluene, and N,N-dimethylformamide.

Further, in step A1, the 1-halododecane includes 1-chlorododecane, 1-bromododecane, or 1-iodododecane, and preferably, the 1-halododecane is 1-bromododecane.

Further, in step A1, the molar ratio of the polyethylene glycol with a single molecular weight to the 1-halododecane is in the range of 1:1 to 15:1 (specifically, such as 1:1, 2:1, 3:1, 4:1, 5:1, 6:1, 7:1, 8:1, 9:1, or 10:1), preferably in the range of 1:1 to 10:1.

Further, in step A1, the molar ratio of the polyethylene glycol with a single molecular weight to the catalyst is in the range of 1:1 to 15:1 (specifically, such as 1:1, 2:1, 3:1, 4:1, 5:1, 6:1, 7:1, 8:1, 9:1, or 10:1), preferably in the range of 1:1 to 10:1.

Further, in step A1, the preset temperature is 80-95 °C (specifically, such as 80 °C, 81 °C, 82 °C, 83 °C, 84 °C, 85 °C, 86 °C, 87 °C, 88 °C, 89 °C, 90 °C, 91 °C, 92 °C, 93 °C, 94 °C, or 95 °C), preferably 80-90 °C.

Further, in step A1, the time for the reaction with the temperature maintained is 12-24 h (specifically, such as 6 h, 17 h, 18 h, 19 h, 20 h, 21 h, 22 h, 23 h, or 24 h), preferably 16-24 h, and more preferably 16-20 h.

Further, in step A2, the extraction operation is repeated 2-6 times (specifically, such as 2, 3, 4, 5, or 6 times), preferably 3-5 times, and more preferably 3 or 4 times.

Further, in step A2, the extraction solvent is selected from one or more of diethyl ether, ethyl acetate, dichloromethane, trichloromethane, methyl tert-butyl ether, and toluene, and more preferably, the extraction solvent is ethyl acetate.

Further, in step A3, the dryer used for the drying is Na₂SO₄ and/or MgSO₄.

Further, in step A3, the separation and purification are performed using a chromatography method, preferably column chromatography, wherein the eluent used in the column chromatography is selected from one or more of ethyl acetate, methanol, ethanol, n-hexane, petroleum ether, and dichloromethane.

Further, in step (2), the solvent is selected from one of ethanol-phosphate buffered saline, ethanol, and normal saline.

In one embodiment of the present disclosure, the solvent is ethanol-phosphate buffered saline with a mass concentration of 4%.

Further, in step (2), the volume ratio of the polidocanol with a single molecular weight to the sodium hyaluronate is in the range of 1:0.1 to 1:5 (specifically, such as 1:0.1, 1:0.2, 1:0.3, 1:0.4, 1:0.5, 1:0.6, 1:0.7, 1:0.8, 1:0.9, 1:1, 1:1.1, 1:1.2, 1:1.3, 1:1.4, 1:1.5, 1:1.6, 1:1.7, 1:1.8, 1:1.9, 1:2, 1:2.2, 1:2.4, 1:2.6, 1:2.8, 1:3, 1:3.2, 1:3.4, 1:3.6, 1:3.8, 1:4, 1:4.2, 1:4.4, 1:4.6, 1:4.8, or 1:5), preferably in the range of 1:0.5 to 1:2, and more preferably 1:1.

Further, in step (2), the mass concentration of the polidocanol with a single molecular weight is 0.01-5% (specifically, such as 0.01%, 0.025%, 0.05%, 0.075%, 0.1%, 0.125%, 0.15%, 0.2%, 0.25%, 0.3%, 0.35%, 0.4%, 0.45%, 0.5%, 1%, 1.5%, 2%, 2.5%, 3%, 3.5%, 4%, 4.5%, or 5%), preferably 0.05-2%, and more preferably 1%.

Further, in step (2), the mass concentration of the sodium hyaluronate is 0.01-5% (specifically, such as 0.01%, 0.025%, 0.05%, 0.075%, 0.1%, 0.125%, 0.15%, 0.2%, 0.25%, 0.3%, 0.35%, 0.4%, 0.45%, 0.5%, 0.75%, 1%, 1.25%, 1.5%, 1.75%, 2%, 2.25%, 2.5%, 2.75%, 3%, 3.25%, 3.5%, 3.75%, 4%, 4.25%, 4.5%, 4.75%, or 5%), preferably 0.05-2%, and more preferably 0.05-0.25%.

Further, in step (2), the volume ratio of the mixed solution to the gas is in the range of 1:0.5 to 1:5 (such as 1:0.5, 1:0.6, 1:0.7, 1:0.8, 1:0.9, 1:1, 1:1.1, 1:1.2, 1:1.3, 1:1.4, 1:1.5, 1:1.6, 1:1.7, 1:1.8, 1:1.9, 1:2, 1:2.2, 1:2.4, 1:2.6, 1:2.8, 1:3, 1:3.2, 1:3.4, 1:3.6, 1:3.8, 1:4, 1:4.2, 1:4.4, 1:4.6, 1:4.8, or 1:5), preferably 1:3.

Further, in step (2), the gas includes air, carbon dioxide, or oxygen, and preferably, the gas is air. Further, in step (2), the method for mixing the mixed solution with the gas to generate bubbles includes the Tessari method, the Tessari/DSS method, or the Monfreux method.

In one embodiment of the present disclosure, the method for mixing the mixed solution with the gas to generate bubbles is the Tessari method.

In a third aspect of the present disclosure, provided is use of the foam sclerosant composition according to the first aspect or a foam sclerosant composition prepared by the preparation method for a foam sclerosant composition according to the second aspect in preparing a medicament for treating a disease.

Further, the disease is selected from one or more of a vascular-related disease, a cystic disease, a tumor, a gynecological and obstetrical disease, an inflammation, and a pain.

Preferably, the vascular-related disease is selected from one or more of varicose veins and vascular malformations.

More preferably, the varicose veins are selected from one or more of varicose veins of the lower extremities, esophagogastric varicose veins, varicocele, abdominal wall varicose veins, and hemorrhoids.

Preferably, the cystic disease includes one or more of an abdominal organ cyst, a body surface cyst, a lymphatic cyst, a thyroid cyst, a bone cyst, a digital mucous cyst, or a pelvic encapsulated effusion. More preferably, the abdominal organ cyst is selected from one or more of a hepatic cyst, a renal cyst, a parapelvic cyst, a pancreatic cyst, an endometrial cyst, and an ovarian chocolate cyst.

More preferably, the body surface cyst includes a skin cyst and/or a digital mucous cyst. Preferably, the tumor includes a benign or malignant solid tumor.

More preferably, the tumor is selected from one or more of infantile hemangioma, congenital hemangioma, tufted hemangioma, spindle cell hemangioma, epithelioid hemangioma, pyogenic granuloma, kaposiform hemangioendothelioma, retiform hemangioendothelioma, papillary intralymphatic hemangioendothelioma, composite hemangioendothelioma, kaposi sarcoma, angiosarcoma, epithelioid hemangioendothelioma, hysteromyoma, and a malignant abdominal organ tumor.

More preferably, the malignant abdominal organ tumor includes liver cancer.

Preferably, the gynecological and obstetrical disease includes uterine submucosal myoma and/or cesarean scar pregnancy.

Preferably, the inflammation includes an infectious inflammation and/or a non-infectious inflammation.

More preferably, the inflammation is selected from one or more of tendinitis, tendonitis, tendon degeneration, peritendinitis, ankylosing spondylitis, sacroiliitis, gouty arthritis, myofasciitis, tenosynovitis, and scapulohumeral periarthritis.

Preferably, the pain includes chronic pain, acute pain, and cancer pain.

More preferably, the pain is selected from one or more of muscle and soft tissue pain, osteoarticular pain, and visceral pain.

The present disclosure has the following beneficial effects:
(1) The preparation process of polidocanol in the present disclosure overcomes the defects of the existing processes, such as numerous impurities in the lauryl alcohol raw material, numerous by-products, and high requirements for process conditions (e.g., pressure and temperature) and equipment, has the advantages of stability and controllable quality, and is suitable for industrial production.
(2) The polidocanol with a single molecular weight and the foamed formulation thereof of the present disclosure, due to the removal of polidocanol with inappropriate PEG chain lengths, exhibit superior foaming effect and foam retention time compared to the foamed formulations of polidocanol in the prior art.
(3) The foam sclerosant of the present disclosure combines polidocanol with a single molecular weight with sodium hyaluronate at a certain concentration. Compared to the foamed formulation of polidocanol with a single molecular weight described above and the hyaluronic acid-polidocanol sclerosant compositions in the prior art, the foam sclerosant of the present disclosure demonstrates higher foam stability, better drug effect, and reduced toxic and side effects.
(4) The polidocanol with a single molecular weight, the foamed formulation thereof, and the foam sclerosant composition of the present disclosure can be widely applied to the treatment of vascular-related diseases, cystic diseases, tumors, gynecological and obstetrical diseases, inflammations, or pains.

### BRIEF DESCRIPTION OF THE DRAWINGS

FIG. 1 shows the nuclear magnetic hydrogen spectrum of DD-PEG8-OH;
FIG. 2 shows the HPLC chromatogram of DD-PEG8-OH;
FIG. 3 shows the chromatogram of the 1% control of DD-PEG8-OH;
FIG. 4 shows the mass spectrum of DD-PEG8-OH;
FIG. 5 shows the nuclear magnetic hydrogen spectrum of DD-PEG9-OH;
FIG. 6 shows the HPLC chromatogram of DD-PEG9-OH;
FIG. 7 shows the chromatogram of the 1% control of DD-PEG9-OH;
FIG. 8 shows the mass spectrum of DD-PEG9-OH;
FIG. 9 shows the nuclear magnetic hydrogen spectrum of DD-PEG10-OH;
FIG. 10 shows the HPLC chromatogram of DD-PEG10-OH;
FIG. 11 shows the chromatogram of the 1% control of DD-PEG10-OH;
FIG. 12 shows the mass spectrum of DD-PEG10-OH.

### DETAILED DESCRIPTION

Unless otherwise defined, all scientific and technical terms used in the present disclosure have the same meaning as commonly understood by those skilled in the art to which the present disclosure relates.

In the present disclosure, the term "single molecular weight" means that the compound of the present disclosure has a single chain length and a precise chemical structure, and it is not formed by mixing homologs with different molecular weights, indicating that the molecular weight of the compound of the present disclosure is not the average molecular weight of a high molecular polymer formed by mixing homologs with different molecular weights. For example, when the polidocanol with a single chain length of the present disclosure contains PEG8 (eight ethylene glycol units), it indicates that the polidocanol with a single chain length contains only PEG8 and does not include polidocanol containing other PEGs such as PEG3, PEG4, PEG5, PEG6, PEG7, PEG9, PEG10, PEG11, PEG12, PEG13, PEG14, PEG15, PEG16, PEG17, PEG18, PEG19, PEG20, PEG21, PEG22, PEG23, PEG24, PEG25, PEG26, PEG27, PEG28, PEG29, PEG30, PEG31, PEG32, PEG33, PEG34, PEG35, PEG36, PEG37, PEG38, PEG39, PEG40, PEG41, PEG42, PEG43, PEG44, PEG45, PEG46, PEG47, PEG48, PEG49, PEG50, and PEG51. The cases for "polidocanol with a single chain length contains PEG9" and "polidocanol with a single chain length contains PEG10" can be understood similarly.

In the present disclosure, the term "Tessari method", also known as the vortex technique, refers to the use of two disposable plastic syringes to generate sclerosing foam. One syringe contains a liquid sclerosant solution, and the other syringe contains gas. The ports of the two syringes are connected at a 90° angle via a three-way stopcock. The contents of the two syringes are rapidly pushed and pulled back and forth 20 times, and after the first 10 pushes, the passage opening is closed as much as possible, generating foam through the resulting turbulence.

In the present disclosure, the term "Tessari/DSS method", also known as the Tessari/double-syringe set technique, is based on the basic Tessari method and uses two latex-free disposable plastic syringes to generate foam. One of the syringes is equipped with a rubber piston. One syringe contains a liquid sclerosant solution, and the other syringe contains gas. The ports of the two syringes are connected in a 180° alignment via a two-way connector. The contents of the two syringes are rapidly pushed and pulled back and forth 5 times (generating additional pressure by firmly holding the piston of one syringe), and then the pushing action is repeated 7 more times (without additional pressure).

In the present disclosure, the term "Monfreux method" refers to a method for generating foam using a glass syringe containing a liquid sclerosant solution. The syringe port is sealed with a rubber cap or plastic cap. The syringe piston is retracted to create a negative pressure, and air is drawn into the syringe through the gap between the syringe barrel and the piston, thereby generating liquid foam containing large bubbles.

In the present disclosure, the term "loading" refers to the operation of adding the product to be separated onto the chromatographic column.

In the present disclosure, "normal-phase separation" refers to the separation of the product using column chromatography in the present disclosure, wherein the stationary phase is polar, and the mobile phase is a solvent less polar than the stationary phase.

In the present disclosure, the term "retention time" refers to the time from the start of sample injection of a separated sample component until the maximum concentration of the component is detected, i.e., the time from the start of sample injection until the apex of the chromatographic peak of a specific component appears. This time is referred to as the retention time of that component, denoted as RT, and is commonly expressed in minutes (min).

In the present disclosure, the term "TLC" refers to thin layer chromatography (TLC), which generally refers to a liquid chromatography method where an adsorbent serves as the stationary phase. This involves uniformly coating the stationary phase as a thin layer on a smooth surface, such as glass, metal, or plastic. The sample is applied at one end of the thin layer, and the mobile phase flows through the stationary phase via capillary action, thereby developing the separated substances.

In the present disclosure, the term "eq" refers to "equivalent", which refers to molar equivalent.

In the present disclosure, the term "HO-PEG10-OH" refers to decaethylene glycol.

In the present disclosure, the term "DD-PEG8-OH" refers to polidocanol containing PEG8 (eight ethylene glycol units), also referred to as "P8" in the present disclosure. The cases for "DD-PEG9-OH" and "DD-PEG10-OH" can be understood similarly.

In the present disclosure, the term "foam drainage time (FDT)" refers to the time at which the liquid begins to drain, i.e., the duration for which the sclerosant liquid is visible.

In the present disclosure, the term "foam halflife time (FHT)" refers to the time required for the foam volume to reduce by half, i.e., the time taken for the foam drainage rate to reach 50%.

In the present disclosure, the term "foam coalescence time (FCT)" refers to the time required for the foam to completely disappear, i.e., the duration for which the sclerosant foam is visible.

Embodiments of the present disclosure will be described in detail below with reference to examples, but those skilled in the art will appreciate that the following examples are only illustrative of the present disclosure and should not be construed as limiting the scope of the present disclosure. Experimental procedures without specified conditions in the examples are conducted according to conventional conditions or conditions recommended by the manufacturer. Reagents or instruments without specified manufacturers used herein are conventional products that are commercially available.

### Example 1. Synthesis of Polidocanol with Single Molecular Weight Having Different Chain Lengths

### 1.1. Synthesis, separation, purification, and characterization of DD-PEG8-OH

NaH (2.2 g, 55 mmol) was added to a 500 mL single-necked flask containing DMSO (200 mL) at room temperature and stirred for 30 min, and octaethylene glycol (20 g, 54.1 mmol) was then added slowly under a nitrogen atmosphere. The mixture was stirred at room temperature for 2 h. 1-Bromododecane (13.5 g, 54.1 mmol) was slowly added to a reaction flask under a nitrogen atmosphere, and the reaction was then heated to 90 °C and stirred for 18 h. The reaction was cooled to room temperature, and water (400 mL) was added. The mixture was extracted 3 times with EtOAc (100 mL). The organic phases were combined, washed with a saturated NaCl solution (100 mL), dried over anhydrous Na₂SO₄, filtered, and concentrated to dryness. The residue was dissolved in DCM and subjected to normal-phase separation with a separation gradient of DCM:MeOH = 0-3%. The product spots were identified with a TLC spotting plate, and the product fractions were combined and concentrated to give a pale yellow solid (2.1 g, yield: 7.2%).

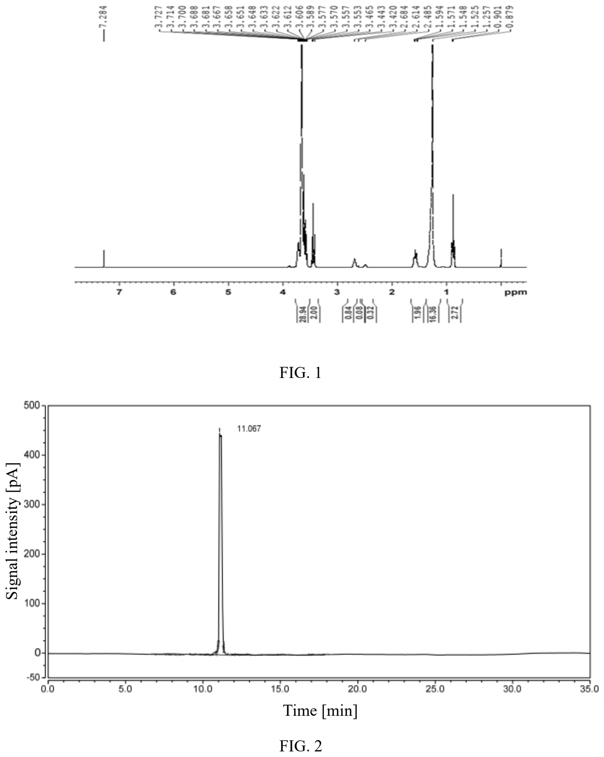

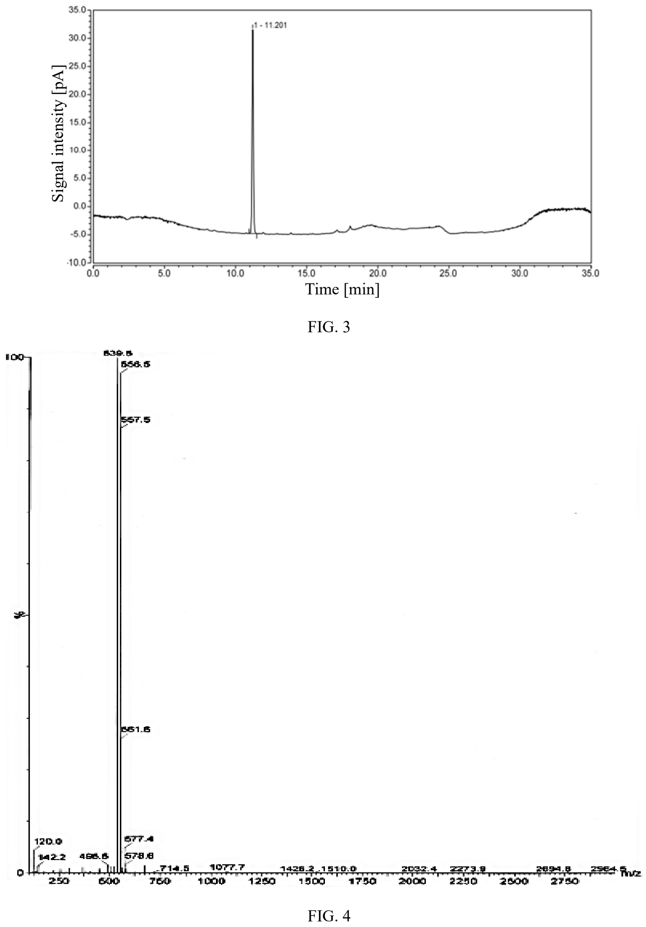

The nuclear magnetic resonance results are shown in FIG. 1. ¹H NMR (300 MHz, CDCl₃) δ: 0.87-0.90 (3H, t), 1.25 (18H, s), 1.52-1.59 (2H, t), 2.68 (1H, s), 3.42-3.46 (2H, t), 3.55-3.72 (29H, m). Using the principal component self-comparison method, the product content was determined to be 98.62%. Specific results are shown in Table 1. The chromatographic results are shown in FIG. 2. FIG. 3 shows the chromatogram of the 1% control sample of DD-PEG8-OH. The mass spectrum results are shown in FIG. 4. MS (ESI+): m/z+H = 539.5.

**Table 1. Chromatogram results of DD-PEG8-OH**

| Sample | Retention time (min) | Area | Total peak area | Content (%) |
|---|---|---|---|---|
| | 6.812 | 0.129 | | 0.03 |
| | 8.087 | 0.6025 | | 0.16 |
| | 8.445 | 0.0167 | | 0 |
| | 8.979 | 0.0381 | | 0.01 |
| | 9.225 | 0.0265 | | 0.01 |
| | 9.56 | 0.1548 | | 0.04 |
| | 9.922 | 0.0593 | | 0.02 |
| | 10.306 | 0.3401 | | 0.09 |
| DD-PEG8-OH | 10.723 | 1.2293 | 386.436 | 0.32 |
| | 11.067 | 381.12 | | 98.62 |
| | 11.68 | 0.1102 | | 0.03 |
| | 11.916 | 0.2002 | | 0.05 |
| | 12.204 | 0.1817 | | 0.05 |
| | 12.936 | 0.1933 | | 0.05 |
| | 13.889 | 0.0447 | | 0.01 |
| | 15.092 | 0.0782 | | 0.02 |
| | 15.398 | 1.3401 | | 0.35 |
| | 17.138 | 0.3049 | | 0.08 |
| | 18.028 | 0.2662 | | 0.07 |
| 1% control | Peak area | 3.8112 | | |

### 1.2. Synthesis, separation, purification, and characterization of DD-PEG9-OH

NaH (484 mg, 12.1 mmol) was added to a 500 mL single-necked flask containing DMSO (200 mL) at room temperature and stirred for 30 min, and nonaethylene glycol (20 g, 48.3 mmol) was then added slowly under a nitrogen atmosphere. The mixture was stirred at room temperature for 2 h. 1-Bromododecane (3.02 g, 12.1 mmol) was slowly added to a reaction flask under a nitrogen atmosphere, and the reaction was then heated to 90 °C and stirred for 18 h. The reaction was cooled to room temperature, and water (400 mL) was added. The mixture was extracted 3 times with ethyl acetate (100 mL). The organic phases were combined, washed with a saturated sodium chloride solution (100 mL), dried over anhydrous Na₂SO₄, filtered, and concentrated to dryness. The residue was dissolved in DCM and subjected to normal-phase separation with a separation gradient of DCM/MeOH (0-3%). The product spots were identified with a TLC spotting plate, and the product fractions were combined and concentrated to give a pale yellow solid (1.36 g, yield: 19.3%).

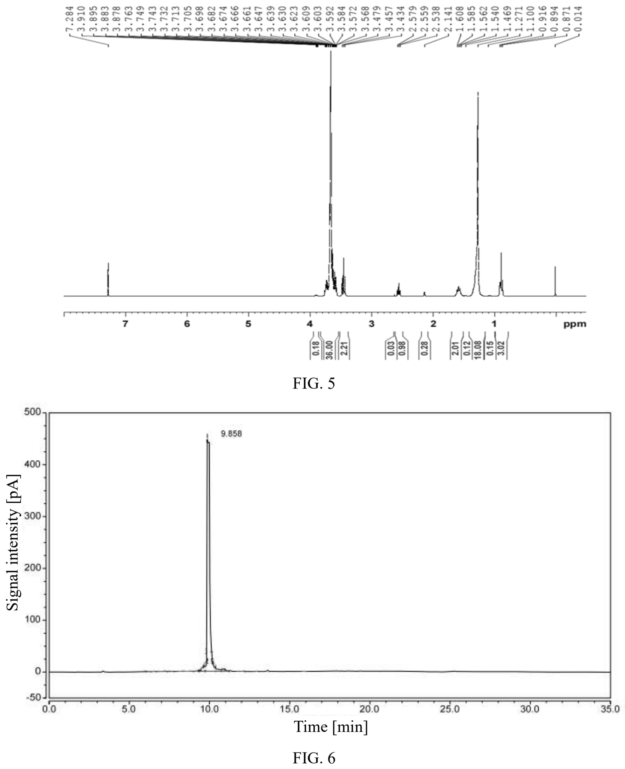

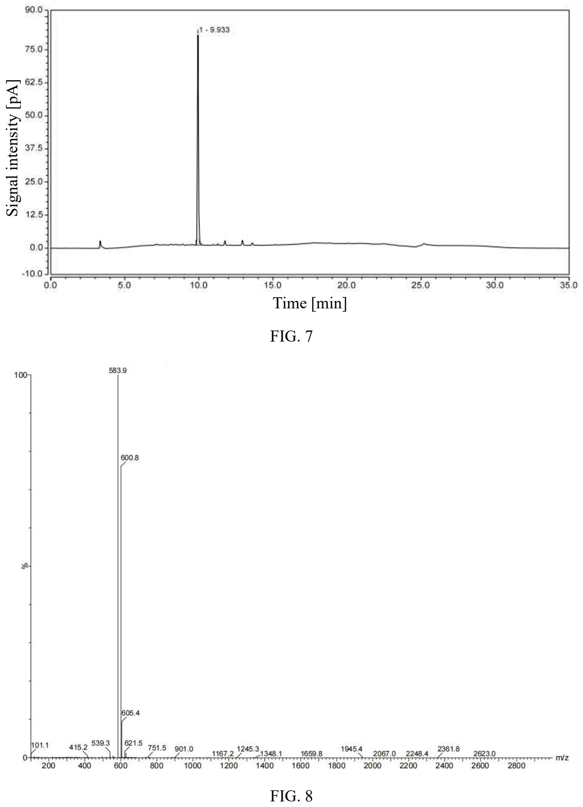

The nuclear magnetic resonance results are shown in FIG. 5. ¹H NMR (300 MHz, CDCl₃) δ: 0.87-0.91 (3H, t), 1.27 (18H, s), 1.56-1.60 (2H, t), 2.53-2.57 (1H, t), 3.43-3.47 (2H, t), 3.56-3.76 (36H, m). The chromatogram results of DD-PEG9-OH are shown in Table 2. The chromatogram is shown in FIG. 6. FIG. 7 shows the chromatogram of the 1% control sample of DD-PEG9-OH. The mass spectrum results are shown in FIG. 8. MS (ESI+): m/z+H = 582.8.

**Table 2. Chromatogram results of DD-PEG9-OH**

| Sample | Retention time | Area | Total peak area | Content (%) |
|---|---|---|---|---|
| | 6.0 | 0.1001 | | 0.02 |
| | 6.129 | 0.0895 | | 1.000 |
| | 6.535 | 0.2639 | | 0.040 |
| | 7.212 | 0.4353 | | 0.070 |
| | 7.573 | 0.08 | | 0.010 |
| | 8.525 | 1.3068 | | 0.20 |
| DD-PEG9-OH | 9.2 | 0.6885 | 666.48 | 0.10 |
| | 9.534 | 2.7165 | | 0.410 |
| | 9.858 | 657.9 | | 98.710 |
| | 10.387 | 1.2469 | | 0.190 |
| | 10.855 | 0.6371 | | 0.100 |
| | 10.970 | 0.4233 | | 0.06 |
| | 12.093 | 0.3367 | | 0.050 |
| | 15.875 | 0.258 | | 0.04 |
| 1% control | Peak area | | 6.579 | |

### 1.3. Chemical synthesis, separation, purification, and characterization of DD-PEG10-OH

HO-PEG10-OH (18 g, 40 mmol, 2 eq) was added to THF (144 mL, 8 V), and NaH (60%, 1.2 g, 1.5 eq) was added with stirring. The mixture was left to react for 20 min. A solution of 1-bromododecane (5 g, 20 mmol, 1 eq) in THF (10 mL) was added dropwise to the system, and after the completion of the dropwise addition, the system was heated to 80 °C and left to react overnight. After the raw materials were completely consumed as detected by TLC, a saturated NH₄Cl solution was added to adjust the pH of the system to <7. The salts were filtered, and THF was then removed by rotary evaporation. Water (100 mL) was added for dilution, and extraction was performed with DCM (3 × 100 mL). Liquid separation was performed, and the organic phases were combined, dried over anhydrous Na₂SO₄, filtered, concentrated to dryness, and purified by column chromatography (MeOH:DCM = 1:10) to elute a pale yellow solid (14 g, yield: 55.8%).

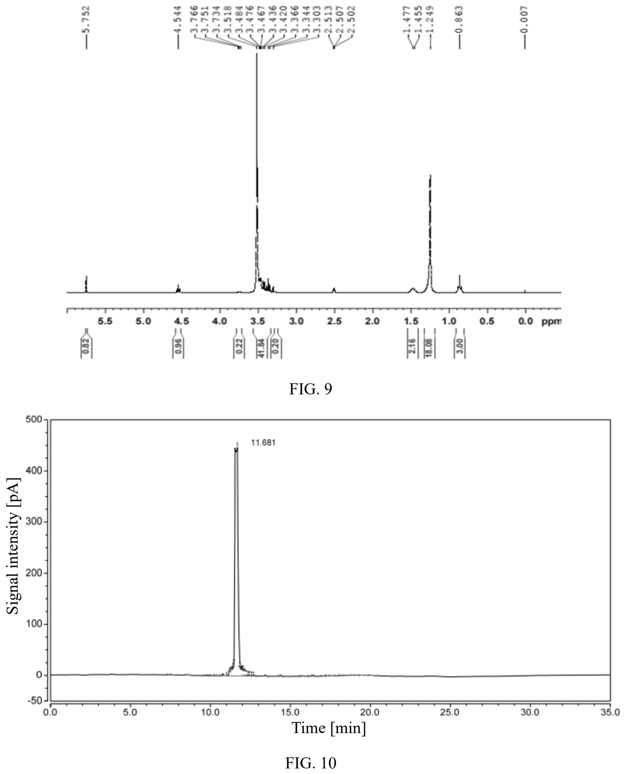

The nuclear magnetic resonance results are shown in FIG. 9. ¹H NMR (300 MHz, CDCl₃) δ: 5.544 (1H, t), 3.518-3.366 (40H, m), 1.477-1.455 (2H, t), 1.249 (18H, s), 0.863 (3H, t).

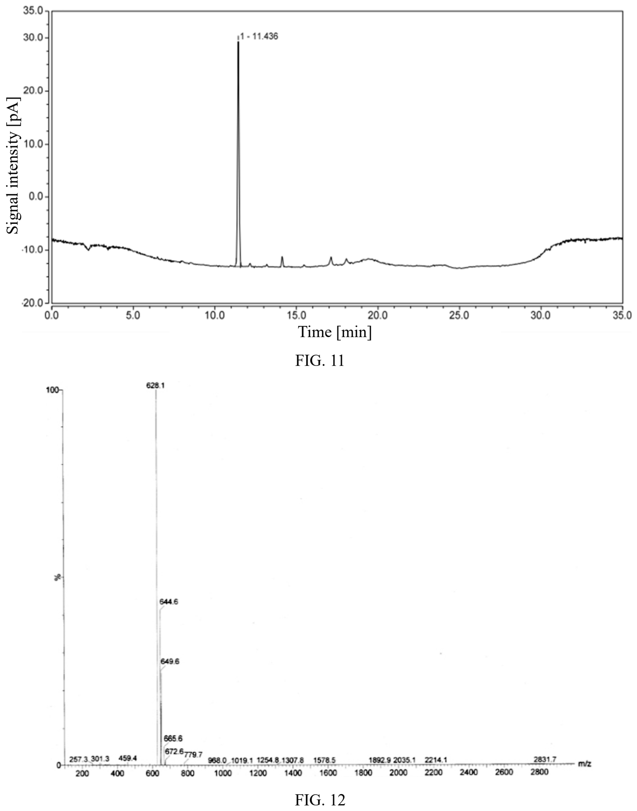

The chromatogram results of DD-PEG10-OH are shown in Table 3. The chromatogram is shown in FIG. 10. FIG. 11 shows the chromatogram of the 1% control sample of DD-PEG10-OH. The mass spectrum results are shown in FIG. 12. MS (ESI+): m/z+H = 628.1.

**Table 3. Chromatogram results of DD-PEG10-OH**

| Sample | Retention time | Area | Total peak area | Content (%) |
|---|---|---|---|---|
| | 7.533 | 0.157 | | 0.03 |
| | 8.582 | 0.393 | | 0.08 |
| | 9.726 | 0.245 | | 0.05 |
| | 9.985 | 0.222 | | 0.05 |
| | 10.367 | 0.111 | | 0.02 |
| | 10.777 | 0.475 | | 0.1 |
| | 11.209 | 2.013 | | 0.43 |
| | 11.681 | 460.6 | | 98.43 |
| | 11.992 | 0.438 | | 0.09 |
| | 12.135 | 0.252 | 467.932 | 0.05 |
| DD-PEG10-OH | 12.381 | 0.34 | | 0.07 |
| | 12.711 | 0.281 | | 0.06 |
| | 13.427 | 0.316 | | 0.07 |
| | 14.358 | 0.345 | | 0.07 |
| | 15.722 | 0.166 | | 0.04 |
| | 16.358 | 0.418 | | 0.09 |
| | 16.469 | 0.038 | | 0.01 |
| | 17.052 | 0.045 | | 0.01 |
| | 17.317 | 0.865 | | 0.18 |
| | 17.793 | 0.05 | | 0.01 |
| | 18.224 | 0.126 | | 0.03 |
| | 18.517 | 0.036 | | 0.01 |
| 1% control | | Peak area | 4.606 | |

### Example 2. Differences in Foam Stability of Polidocanol with Single Molecular Weight Having Different Chain Lengths

### 2.1. Experimental method

Samples of polidocanol with a single molecular weight (DD-PEG8-OH, DD-PEG9-OH, and DD-PEG10-OH prepared in Example 1) and a control (polidocanol in a mixture state containing different molecular weights, purchased from Siegfried Hameln GmbH, Germany, batch number OK13103) were separately weighed and prepared into 1% solutions using a 4% solution of ethanol in PBS. The polidocanol was foamed using the Tessari method, i.e., two 2.5 mL syringes were connected via a three-way tube and rapidly pushed and pulled back and forth at a gas-to-liquid ratio of 3:1 until foam formed. All foam was collected into one syringe, the volume was brought to 2 mL, and the foam state was observed while standing. The following parameters were recorded: foam drainage time, foam halflife time, and foam coalescence time.

### 2.2. Experimental results

P8 represents the polidocanol with a single molecular weight DD-PEG8-OH, P9 represents the polidocanol with a single molecular weight DD-PEG9-OH, and P10 represents the polidocanol with a single molecular weight DD-PEG10-OH.

After thorough mixing with air at a volume ratio of 1:3, the 1% solutions of P8, P9, and P10 all formed homogeneous foam. Their foam drainage time, foam halflife time, and foam coalescence time are shown in Table 4. The following conclusions were drawn:
(1) The foam drainage times for both P8 and P9 samples were 3 s, which was shorter than the foam drainage time for the control (5 s). The foam drainage time for the P10 sample was 8 s, which was slightly longer than the foam drainage time for the control (5 s).
(2) The foam halflife time for the P8 sample was 170 s, which was slightly longer than the foam halflife time for the control (167 s). The foam halflife times for the P9 and P10 samples were 149 s and 158 s, respectively, both of which were shorter than the foam halflife time for the control (167 s).
(3) The foam coalescence times for the P8, P9, and P10 samples were 13 min, 19 min, and 20 min, respectively, all of which were shorter than the foam coalescence time for the control (22 min).

**Table 4. Parameters of P8 P9, P10, and control**

| **Sample concentration** | **Foam drainage time** | **Foam halflife time** | **Foam coalescence time** |
|---|---|---|---|
| P8 (1%) | 3 s | 170 s | 13 m |
| P9 (1%) | 3 s | 149 s | 19 m |
| P10 (1%) | 8 s | 158 s | 20 m |
| Control (1%) | 5 s | 167 s | 22 min |

### Example 3. Effect of Sodium Hyaluronate on Foam Stability of Polidocanol with Single Molecular Weight

### 3.1. Experimental method

The experimental procedures were as follows: (1) A polidocanol sample with a single molecular weight (DD-PEG9-OH prepared in Example 1) was weighed and dissolved in a 4% solution of ethanol in PBS to prepare a 2% solution, which was left to stand until the foam disappeared. (2) A 0.5% sodium hyaluronate solution was prepared and then diluted to concentrations of 0.25%, 0.1%, and 0.05%, respectively. (3) A 2% solution of polidocanol with a single molecular weight was mixed with sodium hyaluronate solutions of different concentration gradients in a volume ratio of 1:1. The concentration of P9 in the solution was made to be 1%, and the concentrations of the sodium hyaluronate solutions were made to be 0.25%, 0.125%, 0.05%, and 0.025%, respectively. (4) A 2.5 mL syringe was used to draw 0.5 mL of the mixed solution, and another syringe was used to draw 1.5 mL of air. Both syringes were connected via a three-way tube, and then the mixture was aspirated 40 times until homogeneous foam was formed. All foam was collected into one syringe, the volume was brought to 2 mL, and the foam state was observed while standing. The following parameters were recorded: foam drainage time, foam halflife time, and foam coalescence time.

### 3.2. Experimental results

As shown in Table 5, the addition of sodium hyaluronate to the 1% P9 solution significantly prolonged the foam halflife time of P9. Within the concentration range of 0.025% to 0.25%, the foam halflife time was positively correlated with the concentration of sodium hyaluronate.

**Table 5. Parameters of P9 and sodium hyaluronate at different concentrations**

| **Sample concentration** | **Foam drainage time** | **Foam halflife time** | **Foam coalescence time** |
|---|---|---|---|
| P9 (1%) | 3 s | 149 s | 19 min |
| P9 (1%) + sodium hyaluronate (0.25%) | 60 s | 684 s | 35 min |
| P9 (1%) + sodium hyaluronate (0.125%) | 90 s | 596 s | 38 min |
| P9 (1%) + sodium hyaluronate (0.05%) | 74 s | 495 s | 38 min |
| P9 (1%) + sodium hyaluronate (0.025%) | 47 s | 312 s | 22 min |
| Control (1%) | 5 s | 167 s | 22 min |

### Example 4. Evaluation of Effect of Hyaluronic Acid Addition on Venous Vascular Sclerosing Effect of Polidocanol with Single Molecular Weight

### 4.1. Experimental method

The rabbit marginal ear vein was used as an experimental model to compare the effects of adding different concentrations of sodium hyaluronate on the venous sclerosing effect of polidocanol. The specific method was as follows: (1) Domestic rabbits were anesthetized with xylazine (2-5 mg/kg, intramuscular injection). Subsequently, the hair on the ears was shaved, and the area was disinfected using a 0.5% chlorhexidine alcohol solution. (2) The Tessari method was used to foam the polidocanol with the addition of sodium hyaluronate, i.e., two 10 mL syringes were connected via a three-way tube and rapidly pushed and pulled back and forth at a certain gas-to-liquid ratio until foam was formed. (3) A disposable syringe was used to puncture the marginal vein, and the sclerosant was injected slowly, with a maximum volume of 0.25 mL. In all groups, manual pressure was applied at the puncture site and along the entire extension of the vein for 90 s. Subsequently, tape was applied at the puncture site. (4) The sclerosing status of the marginal ear veins was observed 3, 7, 14, and 21 days after administration.

### 4.2. Experimental results

### 4.2.1. Results after 14 days of administration in rabbit marginal ear veins

In this example, 14 days after the injection of different types of polidocanol into some rabbit marginal ear veins, some blood vessels were occluded. Visually, this appeared as alternating segments of deep and light color in the blood vessels, and the blood flow in some non-injected blood vessels increased. All rabbit ear blood vessels were scored, with effective ones recorded as 1 and ineffective ones recorded as 0.

The calculation rule for the effective rate was as follows: effective rate = score of rabbit ears/total number of rabbit ears × 100% (calculated based on the number of rabbit ears).

As can be seen from Table 6, 14 days after administration, the positive control group (1% polidocanol, i.e., the control described in Example 2) had an effective rate of 83.33%, and the 1% P9 group (prepared in Example 2) had an effective rate of 91.67%, which was higher than the effective rate of the positive control group; the P9 (1%) + sodium hyaluronate (0.05%) group (prepared in Example 3) had an effective rate of 100%, which was higher than the effective rates of the positive control group and the 1% P9 group. This suggests that the addition of 0.05% sodium hyaluronate to the 1% P9 sample can enhance the *in vivo* vascular sclerosing effect.

**Table 6. Effective rates of venous sclerosis 14 days after injection of different types of polidocanol into rabbit marginal ear veins**

| Group | D14 effective rate (%) |
|---|---|
| Negative control (4% solution of ethanol in PBS) | 0.00 |
| Positive control | 83.33 |
| P9 (1%) | 91.67 |
| P9 (1%) + sodium hyaluronate (0.05%) | 100.00 |
| P9 (0.5%) + sodium hyaluronate (0.05%) | 83.33 |
| P9 (0.25%) + sodium hyaluronate (0.05%) | 66.00 |

### 4.2.2. Results after 21 days of administration in rabbit marginal ear veins

21 days after the injection of different types of polidocanol into some rabbit marginal ear veins, some blood vessels in rabbit ears were completely occluded. Visually, this appeared as some blood vessels becoming lighter in color or gradually disappearing, and the blood flow in non-injected blood vessels increased.

All rabbit ear blood vessels were scored, with effective ones recorded as 1 and ineffective ones recorded as 0. See 4.2.1 for the calculation rule.

The results in Table 7 show that 21 days after administration, the positive control group (1% polidocanol, i.e., the control described in Example 2) had an effective rate of 75%, and the 1% P9 group (prepared in Example 2) had an effective rate of 83.33%, which was higher than the effective rate of the positive control group; the P9 (1%) + sodium hyaluronate (0.05%) group (prepared in Example 3) and the P9 (0.5%) + sodium hyaluronate (0.05%) group (prepared in Example 3) both had an effective rate of 100%, which was higher than the effective rate of the positive control group. This suggests that the addition of 0.05% sodium hyaluronate to the 1% P9 sample can enhance the *in vivo* vascular sclerosing effect.

The P9 (1%) + sodium hyaluronate (0.05%) group and the P9 (0.5%) + sodium hyaluronate (0.05%) group both had an effective rate of 100%. This suggests that the addition of sodium hyaluronate (0.05%) to the P9 sample with a low concentration (0.5%) can achieve the same vascular sclerosing effect as the P9 sample with a high concentration (1%), which helps reduce the usage dose of the P9 sample in clinical applications, thereby reducing toxic reactions and increasing product safety.

**Table 7. Effective rates of venous sclerosis 21 days after injection of different types of polidocanol into rabbit marginal ear veins**

| Group | D21 effective rate (%) |
|---|---|
| Negative control (4% solution of ethanol in PBS) | 0.00 |
| Positive control | 75.00 |
| P9 (1%) | 83.33 |
| P9 (1%) + sodium hyaluronate (0.05%) | 100.00 |
| P9 (0.5%) + sodium hyaluronate (0.05%) | 100.00 |
| P9 (0.25%) + sodium hyaluronate (0.05%) | 83.00 |

Finally, it should be noted that the above examples are only used to illustrate the technical solutions of the present disclosure, but not to limit the present disclosure. Although the present disclosure is described in detail with reference to the examples described above, it will be understood by those of ordinary skill in the art that the technical solutions in the examples described above can still be modified, or some or all of the technical features can be equivalently replaced, and these modifications or replacements do not make the essence of the corresponding technical solutions depart from the scope of the technical solutions in the examples of the present disclosure.

## Claims

1. A foam sclerosant composition, comprising polidocanol and sodium hyaluronate, wherein the polidocanol is polidocanol with a single molecular weight, and a monomer molecular structure thereof comprises the following structure: 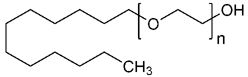 wherein n is an integer, and 4 ≤ n ≤ 24; preferably, 8 ≤ n ≤ 15; more preferably, n is 8, 9, or 10.

2. The foam sclerosant composition according to claim 1, wherein a volume ratio of the polidocanol with a single molecular weight to the sodium hyaluronate is in the range of 1:0.1 to 1:5, preferably in the range of 1:0.5 to 1:2, and more preferably 1:1.

3. The foam sclerosant composition according to claim 1, wherein a mass concentration of the polidocanol with a single molecular weight is 0.01-5%, preferably 0.05-2%, and more preferably 1%.

4. The foam sclerosant composition according to claim 1, wherein a mass concentration of the sodium hyaluronate is 0.01-5%, preferably 0.05-2%, and more preferably 0.05-0.25%.

5. The foam sclerosant composition according to claim 1, further comprising a pharmaceutically acceptable excipient.

6. The foam sclerosant composition according to claim 5, wherein the pharmaceutically acceptable excipient is selected from one or more of a solubilizer, an antioxidant, an anti-photolysis agent, a pH regulator, and an emulsifier;
and/or, a dosage form of the foam sclerosant composition is an injection.

7. A preparation method for the foam sclerosant composition according to claim 1, comprising the following steps:
(1) preparation of polidocanol with a single molecular weight: reacting polyethylene glycol with a single molecular weight with 1-halododecane, separating, and purifying, thereby obtaining the polidocanol with a single molecular weight;
(2) preparation of a foam sclerosant composition: dissolving the polidocanol with a single molecular weight described above in a solvent, allowing the solution to stand, adding sodium hyaluronate, mixing the mixture homogeneously to obtain a mixed solution, and mixing the mixed solution described above with a gas to generate foam, thereby obtaining the foam sclerosant composition.

8. The preparation method according to claim 7, wherein in step (1), the preparation method for the polidocanol with a single molecular weight comprises the following steps:
A1: mixing polyethylene glycol with a single molecular weight with a catalyst and an organic solvent,
adding 1-halododecane dropwise, heating to a preset temperature, and performing reaction with the temperature maintained;
A2: after the reaction is completed, cooling the reactant, and after the reactant is cooled to room temperature, adding water for dilution, followed by extraction and liquid separation to collect an organic phase;
A3: filtering, concentrating, drying, separating, and purifying the organic phase to obtain the polidocanol with a single molecular weight;
and/or, in step A1, the polyethylene glycol with a single molecular weight is selected from tetraethylene glycol, pentaethylene glycol, hexaethylene glycol, heptaethylene glycol, octaethylene glycol, nonaethylene glycol, decaethylene glycol, undecaethylene glycol, dodecaethylene glycol, tridecaethylene glycol, tetradecaethylene glycol, pentadecaethylene glycol, hexadecaethylene glycol, heptadecaethylene glycol, octadecaethylene glycol, nonadecaethylene glycol, eicosaethylene glycol, heneicosaethylene glycol, docosaethylene glycol, tricosaethylene glycol, and tetracosaethylene glycol; preferably, the polyethylene glycol with a single molecular weight is octaethylene glycol, nonaethylene glycol, decaethylene glycol, undecaethylene glycol, dodecaethylene glycol, tridecaethylene glycol, tetradecaethylene glycol, or pentadecaethylene glycol; more preferably, the polyethylene glycol with a single molecular weight is octaethylene glycol, nonaethylene glycol, or decaethylene glycol;
and/or, in step A1, the 1-halododecane comprises 1-chlorododecane, 1-bromododecane, or 1-iodododecane, and preferably, the 1-halododecane is 1-bromododecane;
and/or, in step A1, a molar ratio of the polyethylene glycol with a single molecular weight to the 1-halododecane is in the range of 1:1 to 15:1, preferably in the range of 1:1 to 10:1.

9. The preparation method according to claim 7, wherein in step (2), the solvent is selected from one of ethanol-phosphate buffered saline, ethanol, and normal saline;
and/or, a volume ratio of the polidocanol with a single molecular weight to the sodium hyaluronate is in the range of 1:0.1 to 1:5, preferably in the range of 1:0.5 to 1:2, and more preferably 1:1;
and/or, a mass concentration of the polidocanol with a single molecular weight is 0.01-5%, preferably 0.05-2%, and more preferably 1%;
and/or, a mass concentration of the sodium hyaluronate is 0.01-5%, preferably 0.05-2%, and more preferably 0.05-0.25%;
and/or, a volume ratio of the mixed solution to the gas is in the range of 1:0.5 to 1:5, preferably 1:3;
and/or, the gas comprises air, carbon dioxide, or oxygen, and preferably, the gas is air;
and/or, the method for mixing the mixed solution with the gas to generate bubbles comprises the Tessari method, the Tessari/DSS method, or the Monfreux method.

10. Use of the foam sclerosant composition according to any one of claims 1-6 or a foam sclerosant composition prepared by the preparation method according to any one of claims 7-9 in preparing a medicament for treating a disease, wherein
preferably, the disease is selected from one or more of a vascular-related disease, a cystic disease, a tumor, a gynecological and obstetrical disease, an inflammation, and a pain;
preferably, the vascular-related disease is selected from one or more of varicose veins and vascular malformations;
more preferably, the varicose veins are selected from one or more of varicose veins of the lower extremities, esophagogastric varicose veins, varicocele, abdominal wall varicose veins, and hemorrhoids;
preferably, the cystic disease comprises one or more of an abdominal organ cyst, a body surface cyst, a lymphatic cyst, a thyroid cyst, a bone cyst, a digital mucous cyst, or a pelvic encapsulated effusion; more preferably, the abdominal organ cyst is selected from one or more of a hepatic cyst, a renal cyst, a parapelvic cyst, a pancreatic cyst, an endometrial cyst, and an ovarian chocolate cyst;
more preferably, the body surface cyst comprises a skin cyst and/or a digital mucous cyst; preferably, the tumor comprises a benign or malignant solid tumor;
more preferably, the tumor is selected from one or more of infantile hemangioma, congenital hemangioma, tufted hemangioma, spindle cell hemangioma, epithelioid hemangioma, pyogenic granuloma, kaposiform hemangioendothelioma, retiform hemangioendothelioma, papillary intralymphatic hemangioendothelioma, composite hemangioendothelioma, kaposi sarcoma, angiosarcoma, epithelioid hemangioendothelioma, hysteromyoma, and a malignant abdominal organ tumor;
more preferably, the malignant abdominal organ tumor comprises liver cancer;
preferably, the gynecological and obstetrical disease comprises uterine submucosal myoma and/or cesarean scar pregnancy;
preferably, the inflammation comprises an infectious inflammation and/or a non-infectious inflammation;
more preferably, the inflammation is selected from one or more of tendinitis, tendonitis, tendon degeneration, peritendinitis, ankylosing spondylitis, sacroiliitis, gouty arthritis, myofasciitis, tenosynovitis, and scapulohumeral periarthritis;
preferably, the pain comprises chronic pain, acute pain, and cancer pain;
more preferably, the pain is selected from one or more of muscle and soft tissue pain, osteoarticular pain, and visceral pain.
